# FASCICULE DE BREVET EUROPEEN

(11) **EP 0 761 217 B1**
(45) Date de publication et mention de la délivrance du brevet: **11.03.1998**
(21) Numéro de dépôt: 96401842.8
(22) Date de dépôt: 28.08.1996
(51) Int. Cl.: A61K 31/405, A61K 9/00

(54) **Collyre à base d'indométacine prêt à l'emploi**
Gebrauchsfertige Augentropfen enthaltend Indomethacin
Ready-to-use eye lotion containing indomethacin

(30) Priorité: 06.09.1995 FR 9510450
(43) Date de publication de la demande: 12.03.1997
(73) Titulaire: Laboratoire Chauvin S.A., 34009 Montpellier Cédex 1 (FR)
(72) Inventeur: Maurin, Florence, 34570 Vailhauques (FR); Pages, Bernard, 34560 Montbazin (FR); Coquelet, Claude, 34980 Saint Gely du Fesc (FR)
(74) Mandataire: Le Guen, Gérard

(56) Documents cités:
- EP-A- 0 119 737
- EP-A- 0 244 315
- EP-A- 0 579 435
- CHEMICAL ABSTRACTS, vol. 98, no. 26, 27 Juin 1983 Columbus, Ohio, US; abstract no. 221812, XP002002860 & JP-A-57 200 361 (SUMITOMO CHEMICAL CO. LTD.,JP) 8 Décembre 1982

## Description

La présente invention concerne un collyre à base d'indométacine prêt à l'emploi.

On sait que l'indométacine est un produit pratiquement insoluble dans l'eau et qu'elle subit une hydrolyse en présence d'eau en milieu alcalin.

Pour solubiliser l'indométacine on a déjà préconisé d'utiliser différents tensioactifs mais la stabilité des solutions obtenues n'est pas suffisante (Suleiman et al, Drug Development and Industrial Pharmacy 16, 695, 1990) pour permettre la fabrication de collyres prêts à l'emploi.

Pour résoudre ces difficultés on a proposé d'utiliser des formes lyophilisées contenant des fractions de mélanges tampons qui sont redissoutes dans de l'eau contenant le reste des mélanges tampons au moment de l'emploi (Brevet FR 2 598 081).

On a également décrit des compositions aqueuses contenant de l'indométacine et une cyclodextrine.

Ainsi, EP-A-0 119 737 décrit un hydrogel comprenant de l'indométacine et de la β-cyclodextrine sensiblement dans un rapport molaire 1/1 ainsi que de la L-arginine, et JP-A-57 200 361 décrit une composition du même type avec un rapport molaire β-cyclodextrine/indométacine d'environ 2/1.

On a par ailleurs proposé d'utiliser pour solubiliser des principes actifs instables ou peu solubles dans l'eau des complexes d'inclusion avec des β-cyclodextrines éthérifiées (WO 85/02767), notamment à des pH voisins de 7.

Les β-cyclodextrines éthérifiées sont utilisées dans un rapport molaire principe actif/cyclodextrine de 4/1 à 1/6.

On a maintenant découvert qu'il est possible de solubiliser dans l'eau de l'indométacine et d'obtenir des solutions stables à l'aide de proportions importantes de β ou γ cyclodextrines éthérifiées et en utilisant des pH de 4 à 6. Sans vouloir limiter l'invention il est permis de penser qu'avec de fortes concentrations en β ou γ cyclodextrines éthérifiées on ne forme pas de complexe mais on entoure les molécules d'indométacine de plusieurs noyaux de β ou γ cyclodextrine éthérifiée.

La présente invention a aussi pour objet un collyre d'indométacine prêt à l'emploi comprenant en solution aqueuse
- de l'indométacine
- une β ou γ cyclodextrine éthérifiée par des groupes alkyle en C₁-C₄ ou hydroxyalkyle en C₁-C₄,
ladite cyclodextrine étant présente en un rapport molaire vis à vis de l'indométacine d'au moins 10/1,
et le pH de la solution étant de 4,0 à 6,0.

Les β et γ cyclodextrines éthérifiées sont des composés largement connus et commercialisés. Il s'agit de structures cycliques constituées respectivement par 7 ou 8 motifs d'anhydroglucose. Chacun des motifs glucose contient 3 groupes hydroxy qui sont en partie éthérifiés par des groupes alkyle en C₁-C₄ ou hydroxyalkyle en C₁-C₄. Le degré de substitution par motif d'anhydroglucose varie généralement de 0,05 à 2, notamment de 0,2 à 2.

Dans la présente invention on préfère tout particulièrement l'hydroxypropyl-β-cyclodextrine, mais on peut également utiliser en particulier l'hydroxyethyl-β-cyclodextrine, l'hydroxybutyl-β-cyclodextrine et l'hydroxypropyl-γ-cyclodextrine. Pour obtenir la meilleure stabilité on préfère utiliser les β et γ cyclodextrines éthérifiées, et tout particulièrement l'hydroxypropyl-β-cyclodextrine, dans un rapport molaire d'au moins 25 vis-à-vis de l'indométacine.

Le pH est de préférence ajusté entre 4,5 et 5,5.

Pour préparer les collyres selon l'invention, on peut notamment dissoudre l'indométacine en présence de la β ou γ cyclodextrine éthérifiée dans une solution aqueuse d'un agent alcalin puis ajuster le pH à une valeur de 4 à 6 par addition d'un agent acide.

Comme agent alcalin pour préparer la solution alcaline on peut utiliser notamment l'arginine, le borate de sodium, le citrate de sodium ou l'acétate de sodium.

Comme agent acide on peut utiliser notamment l'acide chlorhydrique, l'acide phosphorique, l'acide borique, l'acide glutamique, l'acide citrique, l'acide acétique, le dihydrogénophosphate de sodium.

Le collyre selon l'invention peut en outre contenir un conservateur antimicrobien tel qu'un dérivé mercuriel, un dérivé de type ammonium quaternaire ou la chlorhexidine.

Les exemples suivants illustrent la présente invention.

### Exemple 1:

On prépare la composition dont la formule est donnée dans le tableau ci-après en dissolvant l'indométacine et l'hydroxypropyl-β-cyclodextrine (ayant un degré de substitution moyen par des groupes hydroxypropyle de 0,4) dans une solution aqueuse d'arginine puis ajustant le pH avec l'acide:

| COMPOSITION | |
|---|---|
| Indométacine | 0,100 g |
| Hydroxypropyl-β-cyclodextrine | 10,00 g |
| Arginine | 0,120 g |
| Acide chlorhydrique concentré sous forme de solution N | qs pH 5,0 |
| Eau purifiée qsp | 100,000 ml |

Le rapport molaire HPβCD/indométacine est d'environ 27,5.

On obtient dans ces conditions une dissolution complète de l'indométacine à 25°C et la solution est stable plusieurs mois.

### Exemple 2:

On opère comme à l'exemple 1 en utilisant les constituants suivants:

| | |
|---|---|
| Indométacine | 0,1 g |
| Hydroxypropyl-γ-cyclodextrine (ayant un degré de substitution moyen de 0,6) | 20,0 g |
| Arginine et acide chlorhydrique 1N qsp | pH = 5,0 |
| Eau distillée qsp | 100,00 ml |

### Exemple comparatif 1 :

On opère comme à l'exemple 1 mais en utilisant la composition suivante.

| | |
|---|---|
| . Indométacine | 0,1 g |
| . βcyclodextrine | 0,5 g |
| . Arginine - Acide chlorhydrique qs | pH = 5,0 |
| . Eau distillée qsp | 100,0 ml |

On observe une recristallisation au cours de la fabrication, c'est à dire qu'il n'est pas possible d'obtenir une solution à 0, 1% (p / v) d'indométacine.

### Essais de stabilité des collyres selon l'invention

1. Stabilité après autoclavage:
On prépare des compositions comme à l'exemple 1 avec différentes concentrations en hydroxypropyl-β-cyclodextrines.

| | |
|---|---|
| . Indométacine | 0,1 g |
| . HPβCD (degré de substitution 0,4) | de 5 à 30 g |
| . Arginine - Acide chlorhydrique qs | pH = 5,0 |
| . Eau purifiée qsp | 100,0 ml |

On étudie la stabilité après autoclavage 20 minutes à 120°C. Les résultats sont donnés dans le tableau suivant:

| Concentrations en HPβCD (g/100ml) | | | | |
|---|---|---|---|---|
| 5 | 10 | 15 | 20 | 30 |
| 97,5% | 100% | 100% | 100% | 100% |

Ce tableau met en évidence l'excellente stabilité des compositions, surtout pour des concentrations à partir de 10 g d'hydroxypropyl-β-cyclodextrine pour 0,1g d'indométacine, soit environ 27,5 fois molaire par rapport à l'indométacine.
2. Stabilité, à long terme à 25 et 40°C
a) On a testé la stabilité de la composition suivante:

| | |
|---|---|
| . Indométacine | 0,1 g |
| . HPβCD (degré de substitution 0,4) | 10,0 g |
| . Arginine - Acide chlorhydrique qs | pH= 5,0 |
| . Eau purifiée qsp | 100,0ml |

Les résultats sont donnés dans le tableau ci-après:

| T = (mois) | Température de conservation | |
|---|---|---|
| | 25°C | 40°C |
| 3 | 99,6% | 97,6% |
| 6 | 99,0% | 94,0% |

b) On a testé la stabilité de la composition de l'exemple 2.
Les résultats sont les suivants au bout de 2 mois.

| Température de conservation | Taux d'indométacine |
|---|---|
| 25°C | 95% |
| 40°C | 94% |

### Essais comparatifs de stabilité à pH7

On a préparé des compositions ayant la formule suivante:

| | |
|---|---|
| . Indométacine | 0,1 g |
| . Hydroxypropyl-β-cyclodextrine (degré de substitution de 0,4) | 0,5 à 50 g |
| . Borate de sodium - acide borique qs | pH7 |
| . Eau purifiée qsp | 100,0 ml |

Les résultats de stabilité à 40°C sont les suivants:

| T (mois) | Concentration en hydroxypropyl-β-cyclodextrine | | | | |
|---|---|---|---|---|---|
| | 0,5 g | 2,5 g | 5 g | 20 g | 50 g |
| 1 | 81,3 % | 89,7 % | 90,7 % | 92,7 % | 93,0 % |
| 2 | 70,7 % | 82,5 % | 85,0 % | 85,0 % | 87,0 % |

Ces résultats montrent que l'hydroxypropyl-β-cyclodextrine ne permet pas d'obtenir une stabilité suffisante même à des concentrations relativement élevées pour des solutions à pH 7.

## Revendications

1. Collyre d'indométacine prêt à l'emploi comprenant en solution aqueuse :
- de l'indométacine,
- une β ou γ cyclodextrine éthérifiée par des groupes alkyle en C₁-C₄ ou hydroxyalkyle en C₁-C₄,
caractérisé en ce que ladite cyclodextrine est présente en un rapport molaire vis-à-vis de l'indométacine d'au moins 10/1,
et le pH de la solution est de 4,0 à 6,0.

2. Collyre selon la revendication 1, dans lequel ladite cyclodextrine est présente en un rapport molaire d'au moins 25 vis-à-vis de l'indométacine.

3. Collyre selon la revendication 1 ou la revendication 2, dans lequel ladite cyclodextrine est l'hydroxypropyl-β-cyclodextrine.

4. Collyre selon la revendication 3, dans lequel l'hydroxypropyl-β-cyclodextrine a un degré de substitution par les groupes hydroxypropyle de 0,4.

5. Collyre selon l'une quelconque des revendications 1 à 4, dans lequel le pH est de 4,5 à 5,5.

## Claims

1. Ready-to-use indomethacin eye lotion comprising, in aqueous solution:
- indomethacin
- a β- or γ-cyclodextrin etherified by C₁-C₄ alkyl or C₁-C₄ hydroxalkyl groups,
characterized in that the said cyclodextrin is present in a molar ratio with respect to the indomethacin of at least 10/1,
and the pH of the solution is from 4.0 to 6.0.

2. Eye lotion according to Claim 1, in which the said cyclodextrin is present in a molar ratio of at least 25 with respect to the indomethacin.

3. Eye lotion according to Claim 1 or Claim 2, in which the said cyclodextrin is hydroxropyl-β-cyclodextrin.

4. Eye lotion according to Claim 3, in which the hydroxypropyl-β-cyclodextrin has a degree of substitution by hydroxypropyl groups of 0.4.

5. Eye lotion according to any one of Claims 1 to 4, in which the pH is from 4.5 to 5.5.

## Patentansprüche

1. Gebrauchsfertiges Augenmittel aus Indometacin umfassend in wäßriger Lösung:
- Indometacin
- ein β oder γ Cyclodextrin, das durch Alkylgruppen an C₁-C₄ oder Hydroxyalkylgruppen an C₁-C₄ verethert ist,
dadurch gekennzeichnet, daß das besagte Cyclodextrin in einem molaren Verhältnis zu dem Indometacin von mindestens 10/1 vorliegt,
und der pH-Wert der Lösung bei 4,0 bis 6,0 liegt.

2. Augenmittel gemäß Anspruch 1, in dem das besagte Cyclodextrin in einem molaren Verhältnis von mindestens 25 zu dem Indometacin vorliegt.

3. Augenmittel gemäß Anspruch 1 oder 2, in dem das besagte Cyclodextrin Hydroxypropyl-β-Cyclodextrin ist.

4. Augenmittel gemäß Anspruch 3, in dem das Hydoxypropyl-β-Cyclodextrin durch die Hydroxypropylgruppen einen Substitutionsgrad von 0,4 besitzt.

5. Augenmittel gemäß einem der Ansprüche 1 bis 4, bei dem der pH-Wert bei 4,5 bis 5,5 liegt.
